(19) Europäisches Patentamt
European Patent Office
Office européen des brevets

(11) Publication number: 0 089 767 B1

(12) # EUROPEAN PATENT SPECIFICATION

(45) Date of publication of patent specification: 11.06.86

(51) Int. Cl.[4]: C 07 D 499/22 // A61K31/43

(21) Application number: 83301199.2

(22) Date of filing: 07.03.83

(54) **Acid addition salts of a penicillanic acid derivative.**

(30) Priority: 19.03.82 US 360035

(43) Date of publication of application: 28.09.83 Bulletin 83/39

(45) Publication of the grant of the patent: 11.06.86 Bulletin 86/24

(84) Designated Contracting States: BE CH DE FR GB IT LI NL SE

(56) References cited:
EP-A-0 004 740
US-A-3 873 521

(73) Proprietor: UPJOHN LIMITED
Fleming Way
Crawley Sussex (GB)

(72) Inventor: Jones, Norman Burnet
c/o Upjohn Limited Fleming Way
Crawley Sussex (GB)

(74) Representative: Perry, Robert Edward et al
GILL JENNINGS & EVERY 53-64 Chancery Lane
London WC2A 1HN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 089 767 B1

Courier Press, Leamington Spa, England.

**Description**

The present invention relates to acid addition salts of a penicillanic acid derivative, i.e. the 6-[D-(2-amino-2-phenylacetamido)]penicillanic acid esters of formula I

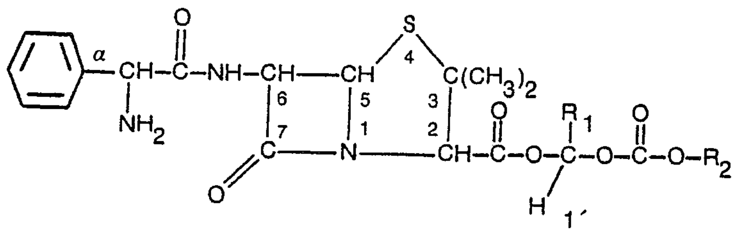

I

wherein $R_1$ is hydrogen, methyl or ethyl and $R_2$ is $C_{1-3}$ alkyl.

Compounds of formula I, and several pharmaceutically acceptable acid addition salts thereof, are disclosed in US—A—3,873,521. The acid addition salts specifically named are the hydrochloride, hydrobromide, hydroiodide, sulfate, citrate, tartrate, maleate, phosphate, acetate, phenoxymethylpenicillinate and tosylate (p-toluenesulfonate). The only one of these for which physiochemical and pharmacological data are given is the hydrochloride. Such salts have an unpleasantly bitter taste.

1-Naphthalenesulfonic acid has the formula

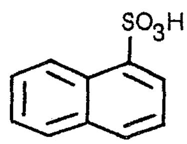

II

US—A—3,180,862 discloses various arylsulfonic acid, including 2-naphthalenesulfonic acid, addition salts of 6-[D-(2-amino-2-phenylacetamido)]penicillanic acid, commonly known as ampicillin, which has the formula

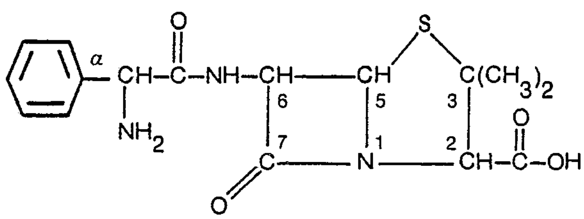

III

EP—A—4740 discloses the improved taste characteristics of the 2-naphthalenesulfonic acid addition salt of the phthalidyl ester of 6-[D-(2-amino-2-phenylacetamido)]penicillanic acid, i.e. the compound of formula I wherein the —$CH(R_1)OCO_2R_2$ moiety is replaced by a phthalidyl moiety.

A novel salt according to the present invention is a 1-naphthalenesulfonic acid addition salt of a compound of formula I, wherein $R_1$ is hydrogen, methyl or ethyl; $R_2$ is $C_{1-3}$ alkyl (i.e. methyl, ethyl, n-propyl or isopropyl); the configurations at the 2-, 5- and 6-positions are as in naturally-occurring benzylpenicillin; the configuration at the α-position is D; and the configuration at the 1′-position, when $R_1$ is methyl or ethyl, is D or L.

A novel crystalline solid according to the present invention is an essentially pure 1-naphthalenesulfonic acid addition salt of a compond of formula I, wherein $R_1$ and $R_2$ are as defined above; the configurations at the 2-, 5- and 6-positions are as in naturally-occurring benzylpenicillin; and the configuration at the α-position is D or L.

A further novel crystalline solid according to the present invention is a mixture of the 1′-position epimers of a compound of formula I, wherein $R_1$ is methyl or ethyl and $R_2$ is $C_{1-3}$ alkyl.

The novel compounds can have utility as broad spectrum antibiotics, and can be useful for treating bacterial infections in mammals, including humans. The antibiotic activity may be due to the formation of ampicillin by rapid hydrolysis of the novel salts after absorption through the mammalian intestinal wall, following introduction into the blood stream or other tissue fluids, e.g. orally.

Surprisingly and unexpectedly, it has been found that the novel salts, in crystalline form, are essentially tasteless in standard taste tests. Corresponding known, pharmaceutically acceptable acid addition salts are unpleasantly bitter in taste. Consequently, the novel salts can be more simply and cheaply formulated into taste-acceptable oral dosage forms, particularly for administration to children.

The compounds of the invention can be prepared, formulated and used, e.g. in the treatment of microbial infections in mammals, as disclosed in US—A—3,873,521, *supra*.

"Crystalline", as used herein, means a solid state which is essentially non-amorphous or essentially non-glassy, i.e. containing no more than about 5% by weight amorphous (glassy) material. "Crystalline solid" or "crystalline salt" includes within its meaning a mass of essentially non-amorphous solid comprising numerous single crystals. The existence of a crystalline or amorphous (glassy) state can be ascertained by numerous techniques, e.g. recording the X-ray diffraction pattern made with a sample of a solid or salt in powdered form.

"Essentially pure", as used herein, means that the material in question exists in a phase with no more than about 5% by weight of other substances, but may include water or another substance if present in a crystalline solid phase as solvent of crystallisation, or a mixture of epimers.

A crystalline solid which comprises the 1-naphthalenesulfonic acid addition salts of a mixture of the position-1' epimers of a compound of formula I wherein $R_1$ is other than hydrogen, regardless of whether only one of the position-1' epimers is present in each single crystal of the mixture or whether both epimers are co-crystallized in some of all the single crystals of the mixture, is considered to comprise a mixture of the salts of both epimers in essentially pure form, provided that no more than a trace of other substances (other than solvent of crystallization) is present in the crystalline solid.

"Alkyl of 1 to 3 carbon atoms" means methyl, ethyl, n-propyl or isopropyl.

Formulas I and III represent compounds which, in salts thereof and at physiological pH's, are at least partially ionized at the amino group bonded to the α-carbon of the phenylacetamido moiety and, in the compound of formula III, at the carboxylate group.

Reference herein to the compound of formula I or formula III includes reference to the ionized forms thereof, to the extent appropriate for the context in which the reference is made.

The preferred salts within the scope of the present invention are the 1-naphthalenesulfonic acid addition salts of both position-1' epimers of the compound of formula I wherein $R_1$ is methyl and $R_2$ is ethyl. The trivial name for a compound of formula I wherein $R_1$ is methyl and $R_2$ is ethyl bacampicillin.

The 1-naphthalenesulfonic acid addition salts of a compound of formula I can be made by combining the compound of formula I and 1-naphthalenesulfonic acid in a solvent such as water, methanol, ethanol, n-propanol, isopropanol or an aqueous solution of any of these alcohols. The salt can then be obtained in either glassy (i.e., amorphous) or essentially pure, crystalline solid form by being precipitated from the solution.

Alternatively, the 1-naphthalenesulfonic acid addition salt of a compound of formula I can be prepared by combining an acid addition salt of the compound of formula I with a salt of 1-naphthalenesulfonic acid in a solvent, such as water, methanol, ethanol, isopropanol, or n-propanol, or an aqueous solution of these alcohols in which the two salts are soluble and at least partially dissociated. The 1-naphthalenesulfonic acid addition salt of the present invention can then be obtained in either glassy (i.e., amorphous) or crystalline solid form, possibly in combination with other substances in the solution, by being precipitated from the solution.

Once obtained in essentially pure but glassy (amorphous) form, a 1-naphthalenefulsonate salt of the present invention can be crystallized by dissolution of the salt in a wam (40—60°C) solvent such as methanol, ethanol, n-propanol, isopropanol or an aqueous solution of the alcohols followed by cooling of the solution to room temperature or below, preferably to 0—10°C.

As illustrated in more detail in Example 1, the preferred method for obtaining the 1-naphthalenesulfonic acid addition salt of a compound of formula I is to add slowly an aqueous methanol solution (50% by volume methanol) of the hydrochloric acid addition salt of the compound of formula I (concentration in the solution: approximately 6—12% by weight) to an aqueous methanol solution (50% by volume methanol) of sodium 1-naphthalenesulfonate (concentration in solution: approximately 3—8% by weight) warmed to 40—55°C and then, after mixture of the solutions is complete, to cool the resulting mixture to between 0 and 10°C. After washing the precipitate, which occurs spontaneously in some cases even before combination of the solutions is completed and in any case with the cooling, with water and then drying it under vacuum at between 35 and 45°C, a crystalline solid comprising the essentially pure 1-naphthalenesulfonic acid addition salt of the compound of formula I is obtained.

1-Naphthalenesulfonic acid and salts thereof are known. See The Merck Index, 9th Ed., Merck & Company, Inc., Rahway, N.J., 1976, Entry 6200.

The compounds of formula I and various pharmaceutically acceptable acid addition salts thereof are also known. U.S. Patent 3,873,521.

The procedures provided above for making the 1-naphthalenesulfonic acid addition salt of a compond of formula I can be used with either position-1' epimer of a compound of formula I, wherein $R_1$ is methyl or ethyl, alone or in any combination with the other position-1' epimer of the compound.

Because the position-1' epimers of a compound of formula I are related as diastereomers, they differ in physical properties. Thus, they can be separated from each other and isolated in pure form by separation procedures (e.g., chromatography) which are based on differences in physical properties. Consequently, the 1-naphthalenesulfonic acid addition salt, in the crystalline solid and other states of either position-1' epimer of a compound of formula I, wherein $R_1$ is methyl or ethyl, can be obtained in essentially pure form without 1-naphthalenesulfonic acid addition salt of the other position-1 epimer.

The salts of the present invention are useful as broad-spectrum antibiotics. In particular, they are useful for treating bacterial infections in mammals, including humans, suffering therefrom.

The 1-naphthalenesulfonic acid addition salts of the present invention can be used to treat bacterial infections in the same way as the compounds of formula I themselves and acid addition salts thereof, as disclosed in U.S. Patent 3,873,521.

The salts of the present invention can be used to treat infections of, for example, non-penicillin-resistant strains of *Proteus mirabilis, Proteus vulgaris, Salmonella typhimurium, Staphylococcus aureus,* and *Streptococcus pyrogenes* in mammals, including humans. They can further be used to treat infections in mammals or any micro-organism against which ampicillin (the compound of formula III) or pharmaceutically acceptable acid addition salts thereof are effective antimicrobially, because the compounds of formula I and pharmaceutically acceptable acid addition salts thereof exert their antibiotic effects in vivo through ampicillin. In the mammalian blood stream and other tissue fluids, compounds of formula I are rapidly converted by hydrolysis to ampicillin, which then acts antimicrobially. For a list of microorganisms against which ampicillin is effective as an antimicrobial agent, see, e.g., Physicians' Desk Reference, 34th Ed., Medial Economics Company, Oradell, N.J. 1980, pp. 1275—1276.

The salts of the present invention are used preferably against Gram-positive bacteria.

A physician or veterinarian of skill is able to ascertain readily, using standard diagnostic techniques, when a person or mammal is suffering from a microbial infection which can be treated with a salt of the present invention and how long such treatment should be continued. The skilled physician or veterinarian is also able to determine readily, for a particular person or mammal suffering from a particular microbial infection susceptible to treatment with a salt of the present invention, what the proper dosage regimen and route of administration are.

The dosage regimen for treating a person or mammal with a salt of the present invention will vary somewhat depending on the compound of formula I which forms the cation of the salt, the severity of the infection, the species and virulence of the infecting microorganism, whether the individual being treated is human or (non-human) mammal and, if mammal, the species thereof, the medical and physical condition and age of the person or mammal being treated, and whether the person or mammal is being treated concurrently or concomitantly with other drugs, including possibly other antimicrobial agents.

Preferably the salts of the present invention are used to treat humans suffering from bacterial infections susceptible to treatment therewith.

For oral administration to a human, the daily dosage will vary from about 50 mg to about 5,000 mg, usually from about 200 mg to about 1200 mg, of 1-naphthalenesulfonate salt, preferably administered in equally divided doses at intervals of 6—12 hours.

Equivalent dosage ranges and schedules can readily be established for administration by routes other than oral and to species of mammal other than human.

Because, as crystalline solids, the salts of the present invention are tasteless while, in solution or glassy (amorphous solid) form, they are slightly bitter or leave slightly unacceptable after-taste, oral administration via dosage forms wherein the salts are present solely or primarily as a crystalline solid is preferred. The most preferred of such dosage forms would be a suspension which comprises a saturated solution of a salt of the invention in the presence of a large excess of salt in crystalline solid form (e.g., at least 95% by weight of the salt in crystalline solid form).

However, the salts can also be administered topically or orally via liquid dosage forms or solid dosage forms wherein the salt is present in solution or a glassy (amorphous solid) form.

Thus the salts of the present invention can be administered via a variety of dosage forms known in the pharmaceutical art and suitable for oral, parenteral or topical administration, including, for example, tablets, pills, capsules, powder packets, solid or liquid suspensions, creams, salves, solutions for topical administration, sterile injectable solutions, and the like. In each dosage form, the salts of the invention will usually be combined with pharmaceutically acceptable diluents or carriers appropriate to the dosage form and known in the pharmaceutical art. These diluents or carriers include, for example, sugars (e.g., sucrose, lactose), starches, gelatin, vegetable oils, water, salts (e.g., sodium chloride, phosphate buffers), alcohols, fatty acid esters, and the like. Flavoring and coloring agents can also be present in the dosage forms. Further, other pharmaceutically active substances which are suitable for simultaneous administration with the antibiotic salts of the present invention may be combined with the salts of the invention in various dosage forms. For example, the salts of the present invention may be combined with other antibiotics in a dosage form. The salts of the present invention will comprise from about 1% to about 100% by weight of the dosage forms through which they are administered.

Description of the Preferred Embodiments

The operation of the present invention is more fully understood by the operation of the following examples.

Example 1

Crystalline Bacampicillin 1-Naphthalenesulfonate

500 gm (2.08 moles) of sodium 1-naphthalenesulfonate is dissolved in a solution of 3.8 l of water and 3.8 l of methanol. The resulting solution is warmed to 50°C. To the warm solution is added, with stirring over a 2 hour period, a solution of 1.00 kg (2.03 moles) of bacampicillin hydrochloride (the hydrochloric acid addition salt of the compound of formula I wherein $R_1$ is methyl and $R_2$ is ethyl) obtained from Astra

Lakemedal AG, S-15185 Sondertale, Sweden, (purity: 94%; 1.91 moles of pure salt) in 4.0 l of water and 4.0 l of methanol. After approximately 2 l of the bacampicillin hydrochloride solution has been added, precipitation begins. After all of the bacampicillin hydrochloride solution is added, the resulting mixture is first allowed to cool to room temperature (approximately 23°C) and then placed in an ice bath and stored at 0°C overnight (approximately 15 hours). The mixture is then filtered, and the solids washed with approximately 3 l of water. The solids are then air-dried for several hours and finally dried overnight (approximately 15 hours) at 40°C in vacuo to yield title product. Yield: 970 gm (1.44 moles), 75.4%. Elemental analysis of the product shows 55.24% C, 5.22% H, 6.27% N, and 9.45% S. The specific rotation of title product dissolved to a concentration of 8.47 gm/l in dimethylformamide at room temperature is +123°. By Karl-Fisher analysis, the title product contains 0.16% water. The NMR spectrum of the title product dissolved in DMSO-$d_6$ exhibits peaks at: 1.21, 1.39, 1.46, 1.50, 2.52, 3.35, 4.16, 4.35, 4.39, 5.10, 5.45, 5.57, 6.68, 7.3—8.05, 8.7 8.85 and 9.34 δ. The NMR data indicate that the title product contains approximately 46 ± 5% of one position-1′ epimer of the bacampicillin cation and approximately 54 ± 5% of the other position-1′ epimer.

Example 2

Antibiotic Potency

The antibiotic potency of the salts of the present invention is illustrated by the data below providing $CD_{50}$'s of the various antibiotic agents, dissolved in inert vehicle, when administered to mice by oral intubation at 0, 24, 48 and 72 hours after challenge of the mice with an interperitoneally administered dose of microorganism which, in the absence of antibiotic agent, would be lethal. The $CD_{50}$ for an agent is the dose at which 50% of the mice are cured under such a dosing schedule. In the data below the first number in each entry is the mean value and the numbers in parenthesis are the 95% confidence limits:

| | $CD_{50}$ (micromoles/kg) | | |
|---|---|---|---|
| Microorganism and Strain | ampicillin | bacampicillin-HCl | bacampicillin 1-naphthalene-sulfonate |
| S. pyrogenes (UC 152) | 0.49 (0.37—0.69) | 0.19 (0.14—0.26) | 0.15 (0.10—0.20) |
| S. aureus | 2.0 (1.3—3.2) | 1.5 (1.0—2.3) | 0.74 (0.43—1.3) |

**Claims**

1. A 1-naphthalenesulfonic acid addition salt of a compound of formula I

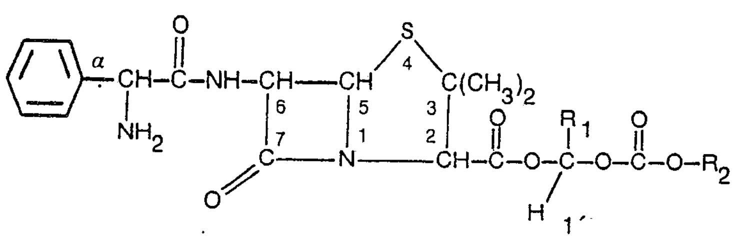

I

wherein $R_1$ is hydrogen, methyl or ethyl; $R_2$ is $C_{1-3}$ alkyl; the configurations at the 2-, 5- and 6-positions as in naturally-occurring benzylpenicillin; the configuration at the α-position is D; and the configuration at the 1′-position, when $R_1$ is methyl or ethyl, is D or L.

2. A salt according to claim 1, wherein $R_1$ is methyl or ethyl and the configuration at the 1′-position is D.

3. A salt according to claim 1, wherein $R_1$ is methyl or ethyl and the configuration at the 1′-position is L.

4. A salt according to any preceding claim, wherein $R_1$ is methyl and $R_2$ is ethyl.

5. A crystalline solid which is an essentially pure 1-naphthalenesulfonic acid addition salt of a compound of formula I as given in claim 1, wherein $R_1$ is hydrogen, methyl or ethyl; $R_2$ is $C_{1-3}$ alkyl; the configurations at the 2-, 5- and 6-positions are as in naturally-occurring benzylpenicillin; and the configuration at the α-position is D; and the configuration at the 1′-position, when $R_1$ is methyl or ethyl, is D or L.

6. A crystalline solid which is a mixture of the essentially pure 1-naphthalenesulfonic acid addition salts fo the 1′-position epimers of a compound of formula I as given in claim 1, wherein $R_1$ is methyl or ethyl and $R_2$ is $C_{1-3}$ alkyl.

7. A crystalline solid according to claim 6, wherein the configuration at the 1′-position is D or L.

8. A crystalline solid according to claim 6 or claim 7, wherein the fraction of each epimer in the mixture is greater than 0.45.

9. A crystalline solid according to any of claims 5 to 8, wherein $R_1$ is methyl and $R_2$ is ethyl.

**Patentansprüche**

1. Ein 1-Naphthalinsulfonsäure-Additionssalz einer Verbindung der Formel I

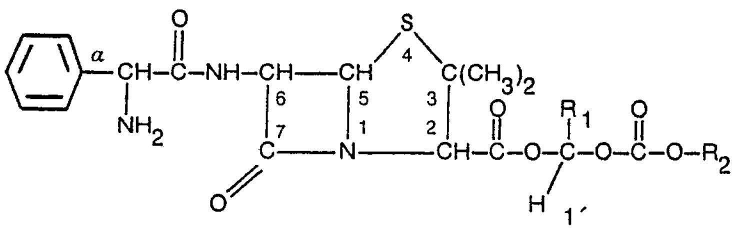

I

bei welcher $R_1$ Wasserstoff, Methyl oder Äthyl ist; $R_2$ $C_{1-3}$ Alkyl ist; die Anordnungen an den 2-, 5- und 6-Stellen wie bei natürlich auftretendem Benzylpenicillin sind; die Anordung an der α-Stelle D ist; und die Anordnung an der 1′-Stelle, falls $R_1$ Methyl oder Äthyl ist, D oder L ist.

2. Ein Salz nach Anspruch 1, bei welchem $R_1$ Methyl oder Äthyl ist und die Anordnung an der 1′-Stelle D ist.

3. Ein Salz nach Anspruch 1, bei welchem $R_1$ Methyl oder Äthyl ist und die Anordnung an der 1′-Stelle L ist.

4. Ein Salz nach einem der vorhergehenden Ansprüche, bei welchem $R_1$ Methyl und $R_2$ Äthyl ist.

5. Ein kristallinischer Feststoff, der aus einem wesentlich reinen 1-Naphthalinsulfonsäure-Additionssalz einer Verbindung der in Anspruch 1 angegebenen Formel I besteht, in welcher $R_1$ Wasserstoff, Methyl oder Äthyl ist; $R_2$ $C_{1-3}$ Alkyl ist; die Anordnungen an den 2-, 5- und 6-Stellen wie bei natürlich auftretendem Benzylpenicillin sind; und die Anordnung an der α-Stelle D ist; und die Anordnung an der 1′-Stelle, falls $R_1$ Methyl oder Äthyl ist, D oder L ist.

6. Ein kristalliner Feststoff aus einem Gemisch wesentlich reiner 1-Naphthalinsulfonsäure-Additionssalze der 1′-Stellung Epimere einer Verbindung der in Anspruch 1 angegebenen Formel I, in welcher $R_1$ Methyl oder Äthyl und $R_2$ $C_{1-3}$ Alkyl ist.

7. Ein kristalliner Feststoff nach Anspruch 6, bei welchem die Anordnung an der 1′-Stelle D oder L ist.

8. Ein kristalliner Feststoff nach Anspruch 6 oder Anspruch 7, bei welchem der Bruchteil jedes der Epimere im Gemisch größer als 0,45 ist.

9. Ein kristalliner Feststoff nach einem der Ansprüche 5 bis 8, in welchem $R_1$ Methyl und $R_2$ Äthyl ist.

**Revendications**

1. Un sel d'addition d'acide 1-naphtalènesulfonique d'un composé de formule I

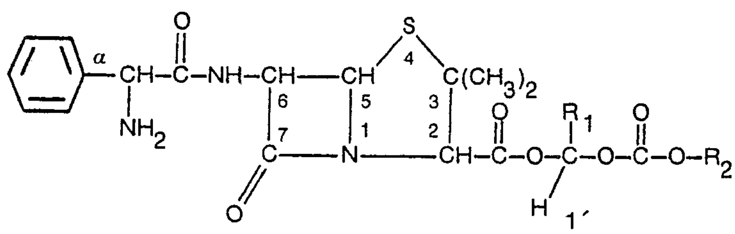

I

dans laquelle $R_1$ est l'hydrogène, le groupe méthyle ou le groupe éthyle; $R_2$ est un groupe alkyle en $C_1$ à $C_3$; les configurations au niveau des positions 2, 5 et 6 sont telles que dans la benzylpénicilline naturelle; la configuration au niveau de la position α est D; et la configuration au niveau de la position 1′, lorsque $R_1$ est le groupe méthyle ou éthyle, est D ou L.

2. Sel suivant la revendication 1, dans lequel $R_1$ est le groupe méthyle ou éthyle et la configuration au niveau de la position 1′ est D.

3. Sel suivant la revendication 1, dans lequel $R_1$ est le groupe méthyle ou éthyle et la configuration au niveau de la position 1′ est L.

4. Sel suivant l'une quelconque des revendications précédentes, dans lequel $R_1$ est le groupe méthyle et $R_2$ est le groupe éthyle.

5. Matière solide cristalline qu est un sel d'addition d'acide 1-naphtalènesulfonique essentiellement pur d'un composé de formule I tel qu'indiqué dans la revendication 1, où $R_1$ est l'hydrogène, le groupe méthyle ou le groupe éthyle; $R_2$ est un groupe alkyle en $C_1$ à $C_3$; les configurations au niveau des positions 2, 5 et 6 sont telles que dans la benzylpénicilline naturelle; la configuration au niveau de la position α est D, et la configuration au niveau de la position 1', lorsque $R_1$ est le groupe méthyle ou éthyle, est D ou L.

6. Matière solide cristalline qui est un mélange des sels d'addition d'acide 1-naphtalènesulfonique essentiellement purs des épimères en position 1' d'un composé de formule I tel qu'indiqué dans la revendication 1, où $R_1$ est le groupe méthyle ou éthyle et $R_2$ est un groupe alkyle en $C_1$ à $C_3$;

7. Matière solide cristalline suivant la revendication 6, dans laquelle la configuration au niveau de la position 1' est D ou L.

8. Matière solide cristalline suivant la revendication 6 ou la revendication 7, dans laquelle la fraction de chaque épimère dans le mélange est supérieure à 0,45.

9. Matière solide cristalline suivant l'une quelconque des revendications 5 à 8, dans laquelle $R_1$ est le groupe méthyle et $R_2$ est le groupe éthyle.